# EUROPEAN PATENT SPECIFICATION

(11) **EP 2 553 147 B1**
(45) Date of publication and mention of the grant of the patent: **18.05.2022**
(21) Application number: 11713569.9
(22) Date of filing: 25.03.2011
(51) Int. Cl.: C25B 11/04, B01J 31/02, G01N 33/00, C25B 1/23, C25B 3/03, C25B 3/07, C25B 3/26, C25B 11/051, C25B 11/095

(54) **NOVEL CATALYST MIXTURES**
NEUE KATALYSATORGEMISCHE
NOUVEAUX MÉLANGES DE CATALYSEUR

(30) Priority: 04.07.2010 US 830338; 26.03.2010 US 317955 P
(43) Date of publication of application: 06.02.2013
(73) Proprietor: Dioxide Materials, Inc., Boca Raton, FL 33431 (US)
(72) Inventor: MASEL, Richard, I., Boca Raton FL 33496 (US); ROSEN, Brian, Wilmington, DE 19810 (US)
(74) Representative: Beyer, Andreas
(86) International application number: PCT/US2011/030098
(87) International publication number: WO 2011/120021

(56) References cited:
- EP-A1- 0 151 510
- EP-A1- 0 323 300
- EP-A2- 0 293 230
- WO-A1-2008/110830
- WO-A1-2009/145624
- CA-A1- 1 272 180
- GB-A- 2 230 782
- US-A- 4 673 473
- US-A- 5 089 661
- US-A- 5 382 332
- US-A1- 2004 031 685
- US-A1- 2008 103 040
- US-A1- 2009 301 297
- US-B2- 7 938 892
- SHUNICHI FUKUZUMI: "Bioinspired Energy Conversion Systems for Hydrogen Production and Storage", EUROPEAN JOURNAL OF INORGANIC CHEMISTRY, vol. 2008, no. 9, 1 March 2008 (2008-03-01), pages 1351-1362, XP055000791, ISSN: 1434-1948, DOI: 10.1002/ejic.200701369
- Anlian Zhu: "Supported choline chloride/urea as a heterogeneous catalyst for chemical fixation of carbon dioxide to cyclic carbonates", Green Chemistry, vol. 9 1 January 2007 (2007-01-01), pages 169-172, XP055000820, Retrieved from the Internet: URL:http://pubs.rsc.org/en/content/article pdf/2007/gc/b612164k
- DERIEN ET AL: "Activation of carbon dioxide: nickel-catalyzed electrochemical carboxylation of diynes", JOURNAL OF ORGANIC CHEMISTRY, vol. 58, no. 9, 1 January 1993 (1993-01-01), pages 2578-2588, XP002108475, AMERICAN CHEMICAL SOCIETY, EASTON.; US ISSN: 0022-3263, DOI: 10.1021/JO00061A038
- DANIEL L. DUBOIS ET AL: "Electrochemical reduction of carbon dioxide catalyzed by [Pd(triphosphine)(solvent)](BF4)2 complexes: synthetic and mechanistic studies", JOURNAL OF THE AMERICAN CHEMICAL SOCIETY, vol. 113, no. 23, 1 November 1991 (1991-11-01), pages 8753-8764, XP055000690, ISSN: 0002-7863, DOI: 10.1021/ja00023a023
- BARBARA J. FISHER ET AL: "Electrocatalytic reduction of carbon dioxide by using macrocycles of nickel and cobalt", JOURNAL OF THE AMERICAN CHEMICAL SOCIETY, vol. 102, no. 24, 1 September 1980 (1980-09-01), pages 7361-7363, XP055000694, ISSN: 0002-7863, DOI: 10.1021/ja00544a035
- THOMAS WELTON: "Room-Temperature Ionic Liquids. Solvents for Synthesis and Catalysis", CHEMICAL REVIEWS, vol. 99, no. 8, 1 January 1999 (1999-01-01), pages 2071-2083, XP002598847, ACS,WASHINGTON, DC, US ISSN: 0009-2665, DOI: 10.1021/CR980032T [retrieved on 1999-07-07]
- MA J ET AL: "A short review of catalysis for CO2 conversion", CATALYSIS TODAY, vol. 148, no. 3-4, 30 November 2009 (2009-11-30), pages 221-231, XP026777534, ELSEVIER, NL ISSN: 0920-5861, DOI: 10.1016/J.CATTOD.2009.08.015 [retrieved on 2009-10-06]
- WENZHONG YANG ET AL: "Electrodeposition of tin and antimony in 1-ethyl- 3-methylimidazolium tetrafluoroborate ionic liquid", JOURNAL OF APPLIED ELECTROCHEMISTRY, KLUWER ACADEMIC PUBLISHERS, DO, vol. 38, no. 4, 25 December 2007 (2007-12-25), pages 537-542, XP019574242, ISSN: 1572-8838
- SESHADRI G ET AL: "A new homogeneous electrocatalyst for the reduction of carbon dioxide to methanol at low overpotential", JOURNAL OF ELECTROANALYTICAL CHEMISTRY AND INTERFACIAL ELECTROCHEMISTRY, ELSEVIER, AMSTERDAM, NL, vol. 372, no. 1-2, 8 July 1994 (1994-07-08), pages 145-150, XP026577882, ISSN: 0022-0728, DOI: 10.1016/0022-0728(94)03300-5 [retrieved on 1994-07-08]
- Buzzeo Marisa C. ET AL: "Voltammetry of Oxygen in the Room-Temperature Ionic Liquids 1-Ethyl-3-methylimidazolium Bis((trifluoromethyl)sulfonyl)imide and Hexyltriethylammonium Bis((trifluoromethyl)sulfonyl)imide:? One-Electron Reduction To Form Superoxide. Steady-State and Transient Behavior in the Same Cyclic Voltammogram R", The Journal of Physical Chemistry A, vol. 107, no. 42, 1 October 2003 (2003-10-01), pages 8872-8878, XP055842090, US ISSN: 1089-5639, DOI: 10.1021/jp0304834 Retrieved from the Internet: URL:https://pubs.acs.org/doi/pdf/10.1021/j p0304834>
- Aoife M. O'mahony ET AL: "The Electrochemical Reduction of Hydrogen Sulfide on Platinum in Several Room Temperature Ionic Liquids", Journal of Physical Chemistry C, vol. 112, no. 20, 22 May 2008 (2008-05-22) , pages 7725-7730, XP055010784, ISSN: 1932-7447, DOI: 10.1021/jp800819k

## Description

### FIELD OF THE INVENTION

The field of the invention is catalysis and catalysts. The catalysts prepared by this invention are applicable to the electrochemical conversion of carbon dioxide into useful products.

### BACKGROUND OF THE INVENTION

There is a present need to decrease carbon dioxide (CO₂) emissions from industrial facilities. Over the years, a number of electrochemical processes have been suggested for the conversion of CO₂ into useful products. Processes for CO₂ conversion and the catalysts for them are discussed in U.S. patents 3,959,094, 4,240,882, 4,523,981, 4,545,872, 4,595,465, 4,608,132, 4,608,133, 4,609,440, 4,609,441, 4,609,451, 4,620,906, 4,668,349, 4,673,473, 4,711,708, 4,756,807, 4,818,353, 5,064,733, 5,284,563, 5,382,332, 5,457,079, 5,709,789, 5,928,806, 5,952,540, 6,024,855, 6,660,680, 6,987,134 (the '134 patent), 7,157,404, 7,378,561, 7,479,570, U.S. patent application 20080223727 (the '727 application) and papers reviewed by Hori (Modern Aspects of Electrochemistry, 42, 89-189, 2008) ("the Hori Review"), Gattrell, et al. (Journal of Electroanalytical Chemistry, 594, 1-19, 2006) ("the Gattrell review"), DuBois (Encyclopedia of Electrochemistry, 7a, 202-225, 2006) ("the DuBois review"), and the papers Li, et al. (Journal of Applied Electrochemistry, 36, 1105-1115, 2006, Li, et al. (Journal of Applied Electrochemistry, 37, 1107-1117, 2007, and Oloman, et al. (ChemSusChem, 1, 385-391, 2008) ("the Li and Oloman papers").

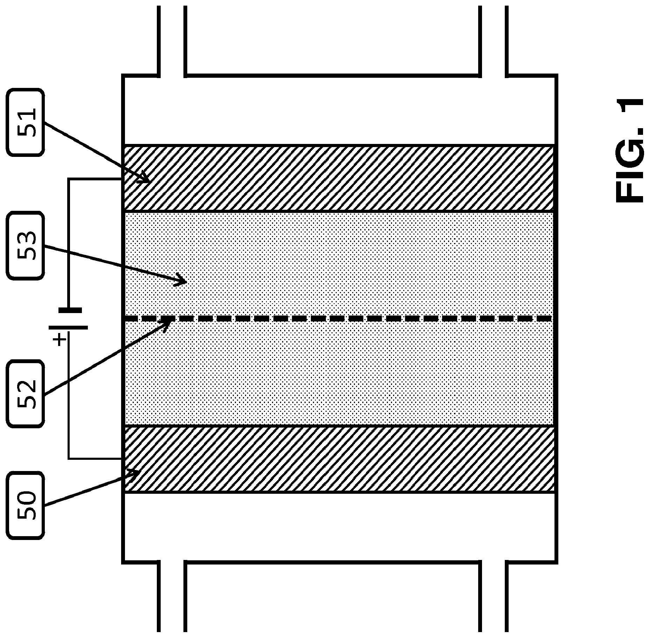

Generally an electrochemical cell contains an anode (50), a cathode (51) and an electrolyte (53) as indicated in Figure 1. Catalysts are placed on the anode, and or cathode and or in the electrolyte to promote desired chemical reactions. During operation, reactants or a solution containing reactants is fed into the cell. Then a voltage is applied between the anode and the cathode, to promote an electrochemical reaction.

When an electrochemical cell is used as a CO₂ conversion system, a reactant comprising CO₂, carbonate or bicarbonate is fed into the cell. A voltage is applied to the cell, and the CO₂ reacts to form new chemical compounds. Examples of cathode reactions in the Hori Review include

CO₂ +2e- → CO + O₂

2CO₂ +2e- → CO + CO₃²⁻

CO₂ + H₂O + 2e- → CO + 2OH⁻

CO₂ + 2H₂O + 4e- → HCO⁻ + 3OH⁻

CO₂ + 2H₂O + 2e- → H₂CO + 2OH⁻

CO₂ + H₂O + 2e- → (HCO₂)⁻ + OH⁻

CO₂ + 2H₂O + 2e- -> H₂CO₂ + 2OH⁻

CO₂ + 6H₂O + 6e- → CH₃OH + 6OH⁻

CO₂ + 6H₂O + 8e- → CH₄ + 8OH⁻

2CO₂ + 8H₂O + 12e- → C₂H₄ + 12OH⁻

2CO₂ + 9H₂O + 12e- → CH₃CH₂OH + 12OH⁻

2CO₂ + 6H₂O + 8e- → CH₃COOH + 8OH⁻

2CO₂ + 5H₂O + 8e- → CH₃COO⁻ + 7OH⁻

CO₂ + 10H₂O + 14e- → C₂H₆ + 14OH⁻

CO₂ + 2H⁺ + 2e → CO + H2O, acetic acid, oxalic acid, oxylate

CO₂ + 4H⁺ + 4e→ CH₄ + O₂

where e- is an electron. The examples given above are merely illustrative and are not meant to be an exhaustive list of all possible cathode reactions.

Examples of reactions on the anode mentioned in the Hori Review include:

2O²⁻ → O₂ + 4e-

2CO₃²⁻ → O₂ + 2CO₂ + 4e-

4OH⁻ → O₂ + 2H₂O + 4e-

2H₂O → O₂ + 4H⁺ + 4e-

The examples given above are merely illustrative and are not meant to be an exhaustive list of all possible anode reactions.

In the previous literature, catalysts comprising one or more of V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Sn, Tl, Pb, Bi, Sb, Te, U, Sm, Tb, La, Ce, and Nd have all shown activity for CO₂ conversion. Reviews include Ma, et al. (Catalysis Today, 148, 221-231, 2009), Hori (Modem Aspects of Electrochemistry, 42, 89-189, 2008), Gattrell, et al. (Journal of Electroanalytical Chemistry, 594, 1-19, 2006), DuBois (Encyclopedia of Electrochemistry, 7a, 202-225, 2006) and references therein.

The results in the Hori Review show that the conversion of CO₂ is only mildly affected by solvent unless the solvent also acts as a reactant. Water can act like a reactant, so reactions in water are different than reactions in non-aqueous solutions. But the reactions are the same in most non-aqueous solvents, and importantly, the overpotentials are almost the same in water and in the non-aqueous solvents.

Zhang, et al. (ChemSusChem, 2, 234-238, 2009) and Chu, et al. (ChemSusChem, 1, 205-209, 2008) report CO₂ conversion catalyzed by an ionic liquid. Zhao, et al. (The Journal of Supercritical Fluids, 32, 287-291, 2004) and Yuan, et al., (Electrochimica Acta 54 (2009) 2912-2915) report the use of an ionic liquid as a solvent and electrolyte, but not a co-catalyst, for CO₂ electroconversion. Catalyst Today Volume 48, pages 189-410 Nov 2009 provides the proceedings of the 10th international conference on CO₂ utilization. The catalysts have been in the form of either bulk materials, supported particles, collections of particles, small metal ions or organometallics. Still, according to Bell (A. Bell. Ed, Basic Research Needs, Catalysis For Energy, US Department Of Energy Report PNNL17712, 2008) ("the Bell Report"), "The major obstacle preventing efficient conversion of carbon dioxide into energy-bearing products is the lack of catalyst" with sufficient activity at low overpotentials and high electron conversion efficiencies.

The overpotential is associated with lost energy of the process, and so one needs the overpotential to be as low as possible. Yet, according to The Bell Report "Electron conversion efficiencies of greater than 50 percent can be obtained, but at the expense of very high overpotentials". This limitation needs to be overcome before practical processes can be obtained.

The '134 patent also considers the use of salt (NaCl) as a secondary "catalyst" for CO₂ reduction in the gas phase, but salt does not lower the overpotential for the reaction.

A second disadvantage of many of the catalysts is that they also have low electron conversion efficiency. Electron conversion efficiencies over 50% are needed for practical catalyst systems.

The examples above consider applications for CO₂ conversion, but the invention overcomes limitations of other systems. For example some commercial CO₂ sensors use an electrochemical reaction to detect the presence of CO₂. At present, these sensors require over 1-5 watts of power, which is too high for portable sensing applications.

Finally, the invention considers new methods to form formic acid. Other methods are discussed in U.S. patents 7,618,725, 7,612,233, 7,420,088, 7,351,860, 7,323,593, 7,253,316, 7,241,365, 7,138,545, 6,992,212, 6,963,909, 6,955,743, 6,906,222, 6,867,329, 6,849,764, 6,841,700, 6,713,649, 6,429,333, 5,879,915, 5,869,739, 5,763,662, 5,639,910, 5,334,759, 5,206,433, 4,879,070, and 4,299,891. These processes do not use CO₂ as a reactant. The article "Electrodeposition of tin and antimony in 1-ethyl-3-methylimidazolium tetrafluoroborate ionic liquid" by Yang et al. (Journal of Applied Electrochemistry, Vol. 38, No. 4 (2007) 537-542) discloses studies on the electrodeposition of Sn(II) and Sb(III) in [EMIm]BF₄ ionic liquid at ambient temperature. The article "A new homogeneous electrocatalyst for the reduction of carbon dioxide to methanol at low overpotential" by Seshadri et al (Journal of Electroanalytical Chemistry, 372 (1994) 145-150) discloses pyridinium ion which is found to be a novel homogeneous catalyst for the reduction of CO₂ to methanol, wherein the reduction of protons to dihydrogen competes with methanol formation, methanol faradaic yields of up to 30% have been observed at hydrogenated Pd electrodes, wherein the former reaction occurs both directly at the electrode surface and also by the reduction of the reduced pyridinium species to form pyridine and hydrogen, wherein the salient feature of this system is that the reduction of CO₂ proceeds at low overpotentials. EP 0 151 510 A1 discloses formate salts of nitrogenous bases containing a tertiary nitrogen atom that are prepared by reacting the nitrogenous base with carbon dioxide and hydrogen in the presence of a solvent and a catalyst comprising an inorganic or organometallic compound of rhodium and an organophosphorus compound, wherein the catalysts disclosed effect the reaction at a lower temperature and at higher productivities relative to the Group VIII metal catalysts of the prior art. US 4,673,473 discloses an apparatus for reducing carbon dioxide to the product includes a reduction cell which has a dual porosity cathode, a catholyte chamber having an inlet, a passageway through which passes an electrolyte, a dual porosity cathode separating the passageway from the catholyte chamber, an anolyte chamber has an inlet and an outlet, wherein a porous anode with a hydrophobic barrier separates the passageway from the anolyte chamber. WO 2008/110830 A1 discloses an electrochemical sensor, the sensor comprising a working electrode; a counter electrode; and an ionic liquid medium extending between the working electrode and the counter electrode, the ionic liquid medium comprising an ionic liquid retained in a support material, wherein the ionic liquid may comprise a cation selected from 1-alkyl-3-methylimidazolium, N-alkylpyridinium, tetraalkylammonium or tetraalkylphosphonium cations and an anion selected from hexafluorophosphate; tetrafluoroborate; trifluoromethylsulfonate; bis[(trifluoromethyl)sulfonyl]amide; trifluoroethanoate; acetate; nitrate, and halides, including fluoride, chloride, and bromide. US 2009/0301297 A1 discloses an article comprising a layered structure having one or more layers and an ionic liquid within the layered structure, wherein the layered structure includes at least one of: a support layer having at least some of the ionic liquid bonded thereto, and configured to reduce the ionic current density of at least one of protons and hydroxyl ions; and a gel containing at least a portion of the ionic liquid. WO 2009/145624 A1 discloses a process for the oxidation of a compound having a hydroxy group, said process comprising the steps of: (a) oxidising said compound having a hydroxy group; (b) reducing carbon dioxide at a cathode; wherein steps (a) and (b) are effected in the presence of an ionic liquid. The article "Voltammetry of Oxygen in the Room-Temperature Ionic Liquids 1-Ethyl-3-methylimidazolium Bis((trifluoromethyl)sulfonyl)imide and Hexyltriethylammonium Bis((trifluoromethyl)sulfonyl)imide: [...]" by Buzzeo et al. (J. Phys. Chem. A, 107 (2003) 8872-8878) discloses the investigation of the electrochemical reduction of oxygen in room-temperature ionic liquids 1-Ethyl-3-methylimidazolium Bis((trifluoromethyl)sulfo-nyl)imide and Hexyltriethylammonium Bis((trifluoromethyl)sulfonyl)imide at a gold microdisk. The article "The Electrochemical Reduction of Hydrogen Sulfide on Platinum in Several Room Temperature Ionic Liquids" by O'Mahony et al. (J. Phys. Chem. C, 112 (2008) 7725-7730) discloses studies on the electrochemical reduction of 1 atm hydrogen sulfide gas at a platinum microelectrode in five room temperature ionic liquids.

### SUMMARY OF THE INVENTION

The invention provides a process for making a chemical reaction product comprising the steps of:
combining a catalytically active element selected from the group consisting of V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Tl, Pb, Bi, Sb, Te, U, Sm, Tb, La, Ce and Nd and a helper catalyst in the presence of a reactant comprising CO2, said helper catalyst comprising 1-ethyl-3-methylimidazonium cations, wherein said helper catalyst further comprises tetrafluoroborate anions;
allowing a reaction to proceed to produce a reaction product, optionally comprising the step of:
   applying electrical energy to the reaction so as to achieve electrochemical conversion of the reactant to a reaction product. When the Catalytically Active Element and the Helper Catalyst are combined, the rate and/or selectivity of a chemical reaction can be enhanced over the rate seen in the absence of the Helper Catalyst. For example, the overpotential for electrochemical conversion of carbon dioxide can be substantially reduced, and the current efficiency (namely, selectivity) for CO₂ conversion can be substantially increased.

The catalytically active element can particularly be selected from the group consisting of Pb, Hg, Tl, In, Cd, Bi, Zr, Cr, Sn, W, Pd and Ru. The reaction product can be at least one of CO, HCO⁻, H₂CO, (HCO₂)⁻, H₂CO₂, CH₃OH, CH₄, C₂H₄, CH₃CH₂OH, CH₃COO⁻, CH₃COOH, C₂H₆, (COOH)₂, and (COO⁻)₂, wherein said reaction product preferably is carbon monoxide (CO) or formic acid (H₂CO₂).

### BRIEF DESCRIPTION OF THE DRAWINGS

Figure 1 is a diagram of a typical electrochemical cell.
Figure 2 is a schematic of how the potential of the system moves as it proceeds along the reaction coordinate in the absence of the ionic liquid if the system goes through a (CO₂)⁻ intermediate. The reaction coordinate indicates the fraction of the reaction that has been completed. A high potential for (CO₂)⁻ formation can create a high overpotential for the reaction.
Figure 3 illustrates how the potential could change when a Helper Catalyst is used. In this case the reaction could go through a CO₂-EMIM complex rather than a (CO₂)⁻, substantially lowering the overpotential for the reaction.
Figures 4a, 4b and 4c illustrate some of the cations that may be used to form a complex with (CO₂)⁻.
Figures 5a and 5b illustrate some of the anions that may help to stabilize the (CO₂)⁻ anion.
Figure 6 illustrates some of the neutral molecules that may be used to form a complex with (CO₂)⁻.
Figure 7 shows a schematic of a cell used for the experiments in Examples 1, 2, 3, 4, and 5.
Figure 8 represents a comparison of the cyclic voltammetry for a blank scan where the catalyst was synthesized as in Example 1 where (i) the EMIM-BF4 was sparged with argon, and (ii) a scan where the EMIM-BF4 was sparged with CO₂. Notice the large negative peak associated with CO₂ complex formation.
Figure 9 represents a series of Broad Band Sum Frequency Generation (BB-SFG) spectra taken sequentially as the potential in the cell was scanned from +0.0 to -1.2 with respect to SHE.
Figure 10 shows a CO stripping experiment done by holding the potential at - 0.6 V for 10 or 30 minutes and them measuring the size of the CO stripping peak between 1.2 and 1.5 V with respect to RHE.
Figure 11 represents a comparison of the cyclic voltammetry for a blank scan where the catalyst was synthesized as in Example 3 where i) the water-choline iodide mixture was sparged with argon and ii) a scan where the water-choline iodide mixture was sparged with CO₂.
Figure 12 shows a comparison of the cyclic voltammetry for a blank scan where the catalyst was synthesized as in Example 4 where i) the water-choline chloride mixture was sparged with argon and ii) a scan where the water-choline chloride mixture was sparged with CO₂.
Figure 13 shows a comparison of the cyclic voltammetry for a blank scan where the catalyst was synthesized as in Example 5 where i) the water-choline chloride mixture was sparged with argon and ii) a scan where the water-choline chloride mixture was sparged with CO₂.
Figure 14 shows a schematic of an example sensor before the Helper Catalyst was added.
Figure 15 shows a schematic of where EMIM BF4 is placed on the sensor.
Figure 16 represents the current measured when the voltage on the sensor was exposed to various gases; the applied voltage on the sensor was swept from 0 to 5 volts at 0.1 V/sec.
Figure 17 represents the resistance of the sensor, in nitrogen and in carbon dioxide. The resistance was determined by measuring the voltage needed to maintain a current of 1 microamp. Time is the time from when the current was applied.

### DETAILED DESCRIPTION OF PREFERRED EMBODIMENT(S)

It is understood that the invention is not limited to the particular methodology, protocols, and reagents, etc., described herein, as these may vary as the skilled artisan will recognize.. It also is to be noted that as used herein and in the appended claims, the singular forms "a," "an," and "the" include the plural reference unless the context clearly dictates otherwise. Thus, for example, a reference to "a linker" is a reference to one or more linkers and equivalents thereof known to those skilled in the art.

Unless defined otherwise, all technical and scientific terms used herein have the same meanings as commonly understood by one of ordinary skill in the art to which the invention pertains. The embodiments of the invention and the various features and advantageous details thereof are explained more fully with reference to the non-limiting embodiments and/or illustrated in the accompanying drawings and detailed in the following description. It should be noted that the features illustrated in the drawings are not necessarily drawn to scale, and features of one embodiment may be employed with other embodiments as the skilled artisan would recognize, even if not explicitly stated herein.

Any numerical value ranges recited herein include all values from the lower value to the upper value in increments of one unit provided that there is a separation of at least two units between any lower value and any higher value. As an example, if it is stated that the concentration of a component or value of a process variable such as, for example, size, angle size, pressure, time and the like, is, for example, from 1 to 90, specifically from 20 to 80, more specifically from 30 to 70, it is intended that values such as 15 to 85, 22 to 68, 43 to 51, 30 to 32 etc., are expressly enumerated in this specification. For values which are less than one, one unit is considered to be 0.0001, 0.001, 0.01 or 0.1 as appropriate. These are only examples of what is specifically intended and all possible combinations of numerical values between the lowest value and the highest value are to be treated in a similar manner.

Moreover, provided immediately below is a "Definitions" section, where certain terms related to the invention are defined specifically. Particular methods, devices, and materials are described, although any methods and materials similar or equivalent to those described herein can be used in the practice or testing of the invention.

### DEFINITIONS

The term "electrochemical conversion of CO2" as used here refers to any electrochemical process where carbon dioxide, carbonate, or bicarbonate is converted into another chemical substance in any step of the process.

The term "CV" as used here refers to a cyclic voltamogram or cyclic voltammetry.

The term "Overpotential" as used here refers to the potential (voltage) difference between a reaction's thermodynamically determined reduction or oxidation potential and the potential at which the event is experimentally observed.

The term "Cathode Overpotential" as used here refers to the overpotential on the cathode of an electrochemical cell.

The term "Anode Overpotential" as used here refers to the overpotential on the anode of an electrochemical cell.

The term "Electron Conversion Efficiency" refers to selectivity of an electrochemical reaction. More precisely, it is defined as the fraction of the current that is supplied to the cell that goes to the production of a desired product.

The term "Catalytically Active Element" as used here refers to any chemical element that can serve as a catalyst for the electrochemical conversion of CO2.

The term "Helper Catalyst" refers to any organic molecule or mixture of organic molecules that does at least one of the following:
(a) Speeds up a chemical reaction or
(b) Lowers the overpotential of the reaction without being substantially consumed in the process.

The term "Active Element, Helper Catalyst Mixture" refers to any mixture that includes one or more Catalytically Active Element(s) and at least one Helper Catalyst

The term "Ionic Liquid" refers to salts or ionic compounds that form stable liquids at temperatures below 200°C.

The term "Deep Eutectic Solvent" refers to an ionic solvent that includes a mixture which forms a eutectic with a melting point lower than that of the individual components.

### SPECIFIC DESCRIPTION

The invention relates generally to a process using Active Element, Helper Catalyst combinations according to claim where the combination does at least one of the following:
Speeds up a chemical reaction, or
Lowers the overpotential of the reaction, without being substantially consumed in the process.

For example such mixtures can lower the overpotential for CO₂ conversion to a value less than the overpotentials seen when the same Catalytically Active Element is used without the Helper Catalyst.

According to the Hori Review, Gattrell, et al. (Journal of Electroanalytical Chemistry, 594, 1-19, 2006), DuBois (Encyclopedia of Electrochemistry, 7a, 202-225, 2006) and references therein, catalysts including one or more of V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Sn, Tl, Pb, Bi, Sb, Te, U, Sm, Tb, La, Ce, and Nd all show activity for CO₂ conversion. Products include one or more of CO, OH⁻, HCO⁻, H₂CO, (HCO₂)⁻, H₂O₂, CH₃OH, CH₄, C2H4, CH₃CH₂OH, CH₃COO⁻; CH₃COOH, C₂H₆, O2, H2, (COOH)2, and (COO⁻)2. Therefore, V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Sn, Tl, Pb, Bi, Sb, Te, U, Sm, Tb, La, Ce, and Nd are each examples of Catalytically Active Elements. Possible products of the reaction include one or more of CO, OH⁻, HCO⁻, H₂CO, (HCO₂)⁻, H₂CO₂, CH₃OH, CH₄, C₂H₄, CH₃CH₂OH, CH₃COO⁻, CH₃COOH, C₂H₆, O₂, H₂, (COOH)₂, and (COO⁻)₂, but the invention is not limited to this list of products.

The Hori Review also notes that Pb, Hg, Tl, In, Cd, Bi, Zr, Cr, Sn and W are best for formic acid production. Furuya, et al. (Journal of Electroanalytical Chemistry, 431, 39-41, 1997) notes that Pd/Ru is also active.

The Hori Review notes that there has been over 30 years of work on the electrochemical conversion of CO₂ into saleable products, but still, according to the Bell Report "Electron conversion efficiencies of greater than 50 percent can be obtained, but at the expense of very high overpotentials". This limitation needs to be overcome before practical processes can be obtained.

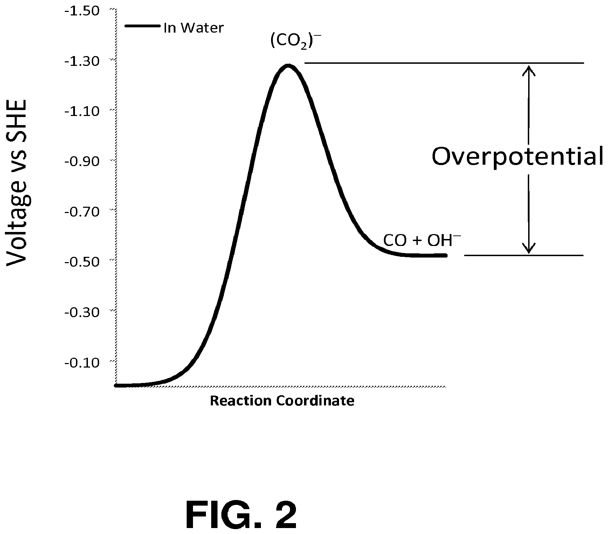

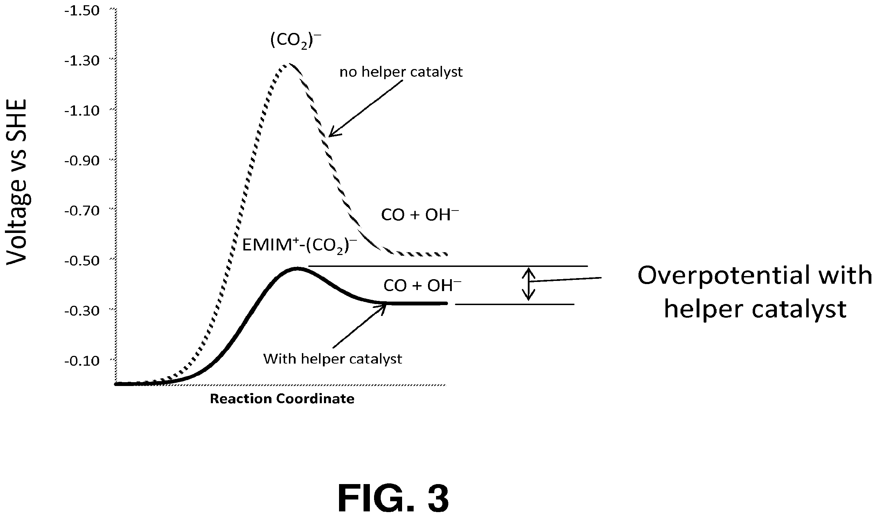

Figures 2 and 3 illustrate one possible mechanism by which a Helper Catalyst can enhance the rate of CO₂ conversion. According to Chandrasekaran, et al. (Surface Science, 185, 495-514, 1987) the high overpotentials for CO₂ conversion occur because the first step in the electroreduction of CO₂ is the formation of a (CO₂)⁻intermediate. It takes energy to form the intermediate as illustrated in Figure 2. This results in a high overpotential for the reaction.

Figure 3 illustrates what might happen if a solution containing I-ethyl-3-methylimidazolium (EMIM⁺) cations is added to the mixture. EMIM⁺ might be able to form a complex with the (CO₂)⁻ intermediate. In that case, the reaction could proceed via the EMIM⁺-(CO₂)⁻ complex instead of going through a bare (CO₂)⁻ intermediate as illustrated in Figure 3. If the energy to form the EMIM⁺-(CO₂)⁻ complex is less than the energy to form the (CO₂)⁻ intermediate, the overpotential for CO₂ conversion could be substantially reduced. Therefore any substance that includes EMIM⁺ cations could act as a Helper Catalyst for CO₂ conversion.

In most cases, solvents only have small effects on the progress of catalytic reactions. The interaction between a solvent and an adsorbate is usually much weaker than the interaction with a Catalytically Active Element, so the solvent only makes a small perturbation to the chemistry occurring on metal surfaces. However, the diagram in Figure 3 shows that such an effect could be large.

Of course a Helper catalyst, alone, will be insufficient to convert CO₂. Instead, one still needs a Catalytically Active Element that can catalyze reactions of (CO₂)⁻ in order to get high rates of CO₂ conversion. Catalysts including at least one of the following Catalytically Active Elements have been previously reported to be active for electrochemical conversion of CO₂:

V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Sn, Tl, Pb, Bi, Sb, Te, U, Sm, Tb, La, Ce, and Nd.

Many of these catalysts also show activity for a number of other reactions. All of these elements are specifically included as Catalytically Active Elements for the purposes of the invention.

Further, those skilled in the art should realize that the diagram in Figure 3 could be drawn for any molecule that could form a complex with (CO₂)⁻. Previous literature indicates that solutions including one or more of: ionic liquids, deep eutectic solvents, amines, and phosphines; including specifically imidazoliums (also called imidazoniums), pyridiniums, pyrrolidiniums, phosphoniums, ammoniums, sulfoniums, prolinates, and methioninates can form complexes with CO₂. Consequently, they may serve as Helper Catalysts. Also Davis Jr., et al. (In ACS Symposium Series 856: Ionic Liquids as Green Solvents: Progress and Prospects, 100-107, 2003) list a number of other salts that show ionic properties. Specific examples include compounds including one or more of acetocholines, alanines, aminoacetonitriles, methylammoniums, arginines, aspartic acids, threonines, chloroformamidiniums, thiouroniums, quinoliniums, pyrrolidinols, serinols, benzamidines, sulfamates, acetates, carbamates, inflates, and cyanides. These salts may act as helper catalysts.

Of course, not every substance that forms a complex with (CO₂)⁻ will act as a helper catalyst. Masel (Chemical Kinetics and Catalysis, Wiley 2001, p717-720), notes that when an intermediate binds to a catalyst, the reactivity of the intermediate decreases. If the intermediate bonds too strongly to the catalyst, the intermediate will become unreactive, so the substance will not be effective. This provides a key limitation on substances that act as Helper Catalysts. The Helper Catalyst cannot form so strong a bond with the (CO₂)⁻ that the (CO₂)⁻ is unreactive toward the Catalytically Active Element.

More specifically, one wishes the substance to form a complex with the (CO₂)⁻ so that the complex is stable (that is, has a negative free energy of formation) at potentials less negative than 1 V with respect to the standard hydrogen electrode (SHE.) However, the complex should not be so stable that the free energy of the reaction between the complex and the Catalytically Active Element is more positive than about 3 kcal/mol

Those trained in the state of the art should realize that the ability of the helper catalyst to stabilize the (CO₂)⁻ also varies with the anion. For example Zhao, et al. (The Journal of Supercritical Fluids, 32, 287-291, 2004) examined CO₂ conversion in l-n-butyl-3-methylimidazolium hexafluorophosphate (BMIM-PF6), but Figure 3 in Zhao, et ah, shows that the BMIM-PF6 did NOT lower the overpotential for the reaction (that is, the BMIM-PF6 did not act as a Helper Catalyst.) This may be because the BMIM-PF6 formed such a strong bond to the (CO₂)⁻ that the CO₂ was unreactive with the copper. Similarly Yuan, et al., Electrochimica Acta 54 (2009) 2912-2915, examined the reaction between methanol and CO₂ in 1-butyl-3-methylimidazolium bromide (BMIM-Br). The BMIM-Br did not act as a Helper Catalyst. This may be because the complex was too weak or that the bromine poisoned the reaction.

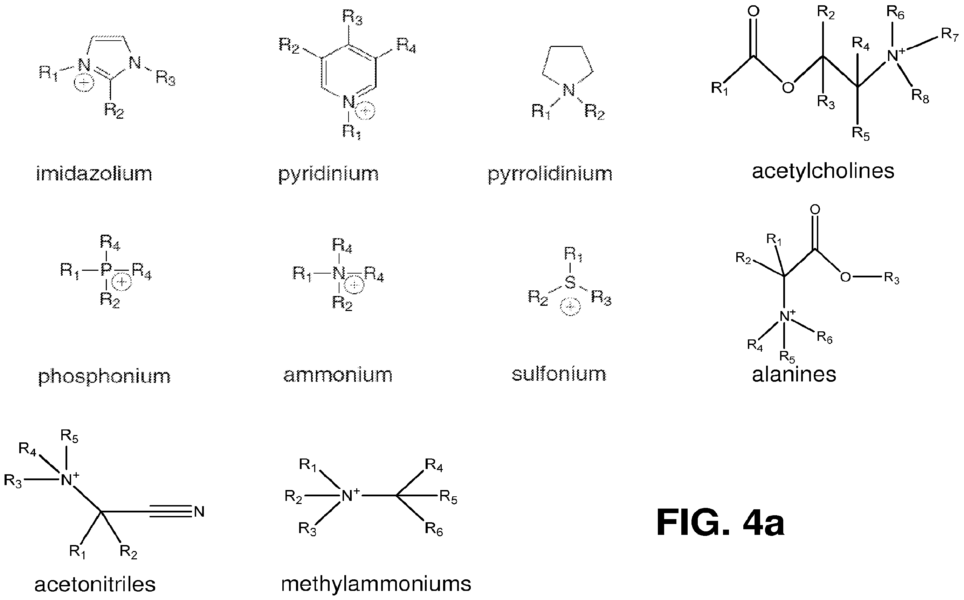

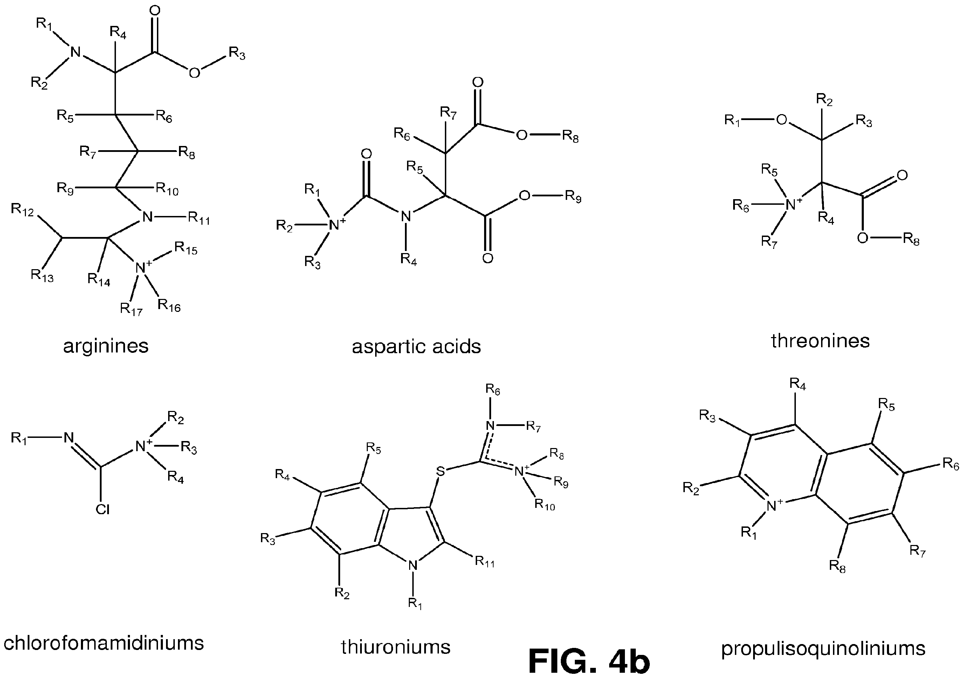

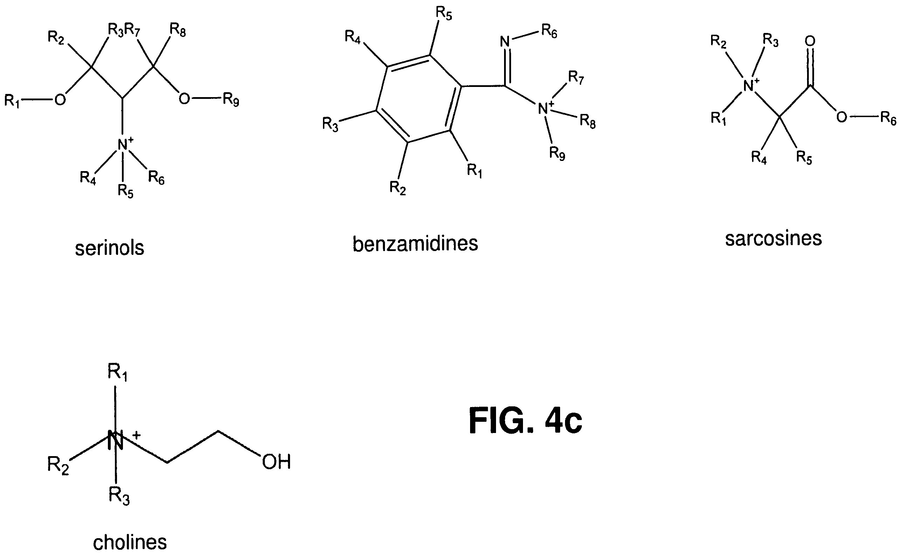

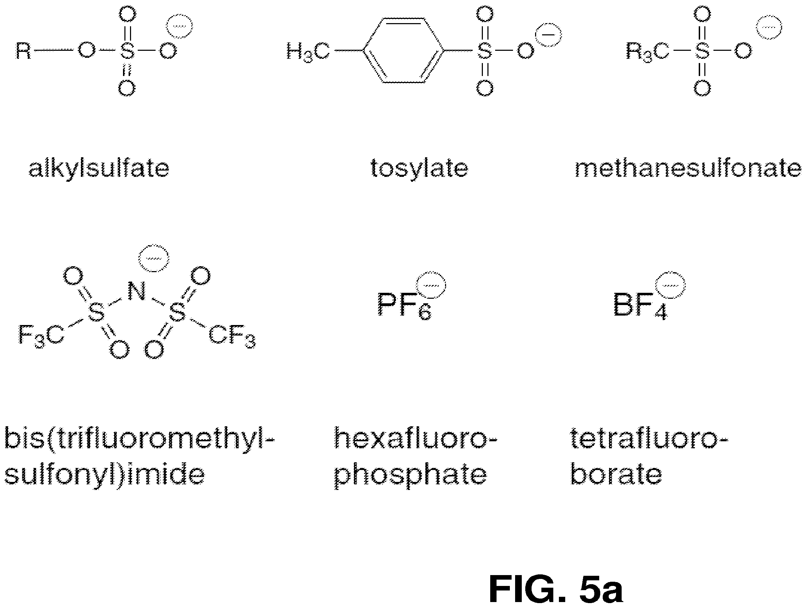

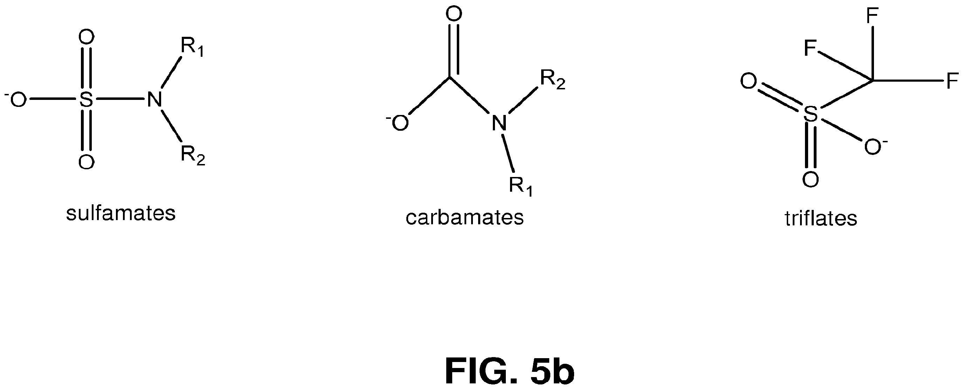

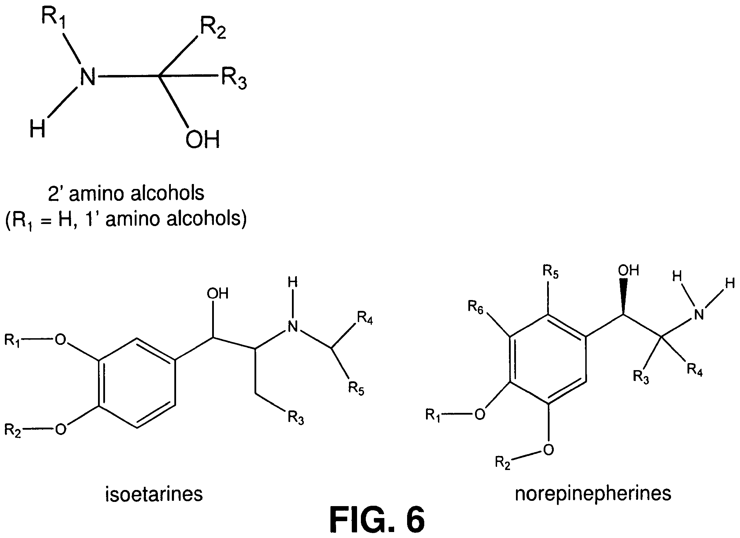

Solutions that include one or more of the cations in Figure 4, the anions in Figure 5, and/or the neutral species in Figure 6, where R1, R2 and R3 (and R4-R17) include H, OH or any ligand containing at least on carbon atom, are believed to form complexes with CO₂ or (CO₂)⁻. Specific examples include: imidazoliums (also called imidazoniums), pyridiniums, pyrrolidiniums, phosphoniums, ammoniums, sulfoniums, prolinates, and methioninates. All of these examples might be able to be used as Helper Catalysts for CO₂ conversion.

In general one can determine whether a given substance S can be a helper catalyst for a reaction R catalyzed by an active metal M as follows:
Fill a standard 3 electrode electrochemical cell with the electrolyte commonly used for reaction R. Common electrolytes include such as 0.1 M sulfuric acid or 0.1 M KOH in water can also be used.

Mount the active metal into the 3 electrode electrochemical cell and an appropriate counter electrode.

Run several CV cycles to clean the active metal.

Measure the reversible hydrogen electrode (RHE) potential in the electrolyte

Load the reactants for the reaction R into the cell, and measure a CV of the reaction R, noting the potential of the peak associated with the reaction R.

Calculate V1 = the difference between the onset potential of the peak associated with reaction and RHE

Calculate V1A = the difference between the maximum potential of the peak associated with reaction and RHE

Add 0.0001 to 99.9999% of the substance S to the electrolyte.

Measure RHE in the reaction with helper catalyst

Measure the CV of reaction R again, noting the potential of the peak associated with the reaction R.

Calculate V2 = the difference between the onset potential of the peak associated with reaction and RHE

Calculate V2A = the difference between the maximum potential of the peak associated with reaction and RHE

If V2<V1 or V2A< V1A at any concentration of the substance S between 0.0001 and 99.9999%, the substance S can be a helper catalyst for the reaction.

Further, the Helper Catalyst could be in any one of the following forms: (i) a solvent for the reaction; (ii) an electrolyte; (iii) an additive to any component of the system; or (iv) something that is bound to at least one of the catalysts in a system and comprises 1-ethyl-3-methylimidazonium cations and tetrafluoroborate anions as defined in the claims.

Those trained in the state of the art should recognize that one might only need a tiny amount of the Helper Catalyst to have a significant effect. Catalytic reactions often occur on distinct active sites. The active site concentration can be very low, so in principle a small amount of Helper Catalyst can have a significant effect on the rate. One can obtain an estimate of how little of the helper catalyst would be needed to change the reaction from Pease, et al., JACS 47, 1235 (1925) study of the effect of carbon monoxide (CO) on the rate of ethylene hydrogenation on copper. Pease, et al., found that 0.05 cc's (62 micrograms) of carbon monoxide (CO) was sufficient to almost completely poison a 100 gram catalyst towards ethylene hydrogenation. This corresponds to a poison concentration of 0.0000062% by weight of CO in the catalyst. Those trained in the state of the art know that if 0.0000062% by weight of the poison in a Catalytically Active Element-poison mixture could effectively suppress a reaction, then as little as 0.0000062% by weight of Helper Catalyst in an Active Element, Helper Catalyst Mixture could enhance a reaction. This provides an estimate of a lower limit to the Helper Catalyst concentration in an Active Element, Helper Catalyst Mixture.

The upper limit is illustrated in Example 1 below, where the Active Element, Helper Catalyst Mixture could have approximately 99.999% by weight of Helper Catalyst, and the Helper Catalyst could be at least an order of magnitude more concentrated. Thus the range of Helper Catalyst concentrations for the invention here may be 0.0000062% to 99.9999% by weight.

Figure 3 only considered the electrochemical conversion of CO₂, but the method is general. There are many examples where energy is needed to create a key intermediate in a reaction sequence. Examples include: homogeneously catalyzed reactions, heterogeneously catalyzed reactions, chemical reactions in chemical plants, chemical reactions in power plants, chemical reactions in pollution control equipment and devices, chemical reactions in safety equipment, chemical reactions in fuel cells, and chemical reactions in sensors. Theoretically, if one could find a Helper Catalyst that forms a complex with a key intermediate, the rate of the reaction should increase. All of these examples are within the scope of the invention.

Specific examples of specific processes that may benefit with Helper Catalysts include the electrochemical process to produce products including one or more of Cl₂, Br₂, I₂, NaOH, KOH, NaClO, NaClO₃, KClO₃, CF₃COOH.

Further, the Helper Catalyst could enhance the rate of a reaction even if it does not form a complex with a key intermediate. Examples of possible mechanisms of action include the Helper Catalyst (i) lowering the energy to form a key intermediate by any means, (ii) donating or accepting electrons or atoms or ligands, (iii) weakening bonds or otherwise making them easier to break, (iv) stabilizing excited states, (v) stabilizing transition states, (vi) holding the reactants in close proximity or in the right configuration to react, or (vii) blocking side reactions. Each of these mechanisms is described on pages 707 to 742 of Masel, Chemical Kinetics and Catalysis, Wiley, NY (2001).

**The** invention includes any process that uses an Active Element, Helper Catalyst Mixture as a catalyst as further defined in the claims. Fuel cells and sensors are useful for understanding the invention.

Without further elaboration, it is believed that one skilled in the art using the preceding description can utilize the invention to the fullest extent.

### Specific Example 1

Using an Active Element, Helper Catalyst Mixture including platinum and 1-ethyl-3-methylimidazolium tetrafluoroborate (EMIM-BF4) to lower the overpotential for electrochemical conversion of CO₂ and raise the selectivity (current efficiency) of the reaction.

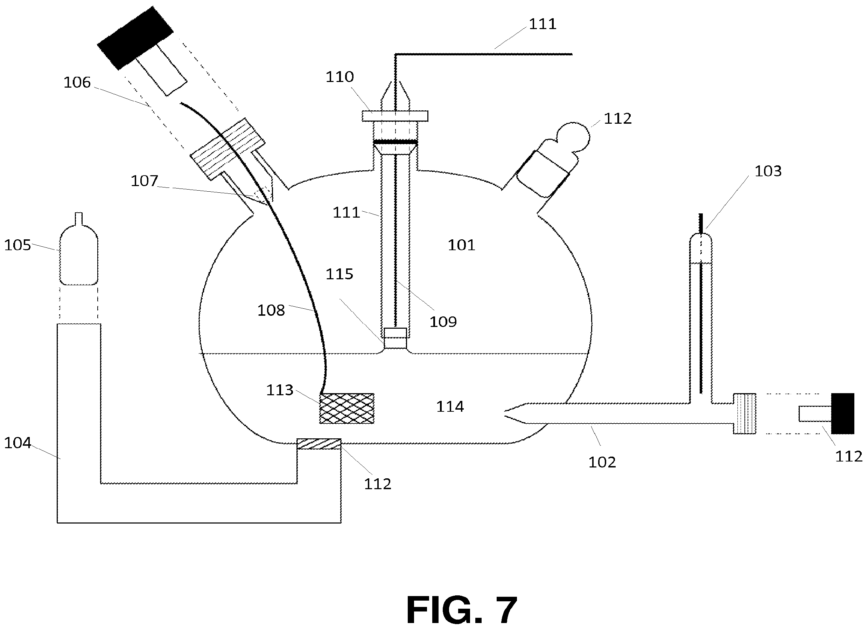

The experiments used the glass three electrode cell shown in Figure 7. The cell consisted of a three neck flask (101), to hold the anode (108), and the cathode (109). A silver/0.01 molar silver ion reference electrode (103) in acetonitrile was connected to the cell through a Luggin Capillary (102). The reference electrode (103) was fitted with a Vycor^{®} frit to prevent any of the reference electrode solution from contaminating the ionic liquid in the capillary. The reference electrode was calibrated against the ferrocene Fc/Fc+ redox couple. A conversion factor of +535 was used to convert our potential axis to reference the Standard Hydrogen Electrode (SHE). A 25x25mm platinum gauze (size 52) (113) was connected to the anode while a 0.33 cm2 polycrystalline gold plug (115) was connected to the cathode.

Prior to the experiments all glass parts were put through a 1% Nochromix^{®} bath (2hrs), followed by a 50/50 v/v nitric acid/water bath (12hrs), followed by rinsing with Millipore water. In addition the gold plug (115) and platinum gauze (113) were mechanically polished using procedures known to workers trained in the art. They were then cleaned in a sulfuric acid bath for 12 hours.

During the experiment a catalyst ink comprising a Catalytically Active Element, platinum, was first prepared as follows: First 0.056 grams of Johnson-Matthey Hispec 1000 platinum black purchased from Alfa-Aesar was mixed with 1 gram of Millipore water and sonicated for 10 minutes to produce a solution containing a 5.6mg/ml suspension of platinum black in Millipore water. A 25 µl drop of the ink was placed on the gold plug (115) and allowed to dry under a heat lamp for 20 min, and subsequently allowed to dry in air for an additional hour. This yielded a catalyst with 0.00014 grams of Catalytically Active Element, platinum, on a gold plug. The gold plug was mounted into the three neck flask (101). Next a Helper Catalyst, EMIM-BF4 (EMD Chemicals, Inc., San Diego, CA, USA) was heated to 120°C under a -23 in. Hg vacuum for 12 hours to remove residual water and oxygen. The concentration of water in the ionic liquid after this procedure was found to be ca. 90mM by conducting a Karl-Fischer titration. (That is, the ionic liquid contained 99.9999% of Helper Catalyst.) 13 grams of the EMIM-BF4 was added to the vessel, creating an Active Element, Helper Catalyst Mixture that contained about 99.999% of the Helper Catalyst. The geometry was such that the gold plug formed a meniscus with the EMIM-BF4 Next ultra-high-purity (UHP) argon was fed through the sparging tube (104) and glass frit (112) for 2 hours at 200 sccm to further remove any moisture picked up by contact with the air.

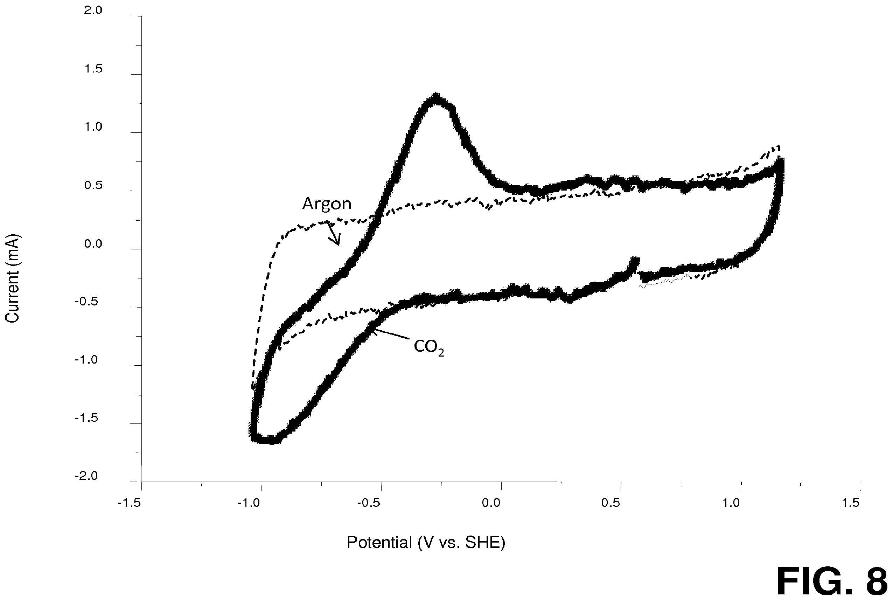

Next the cathode was connected to the working electrode connection in an SI 1287 Solartron electrical interface, the anode was connected to the counter electrode connection and the reference electrode was connected to the reference electrode connection on the Solartron. Then the potential on the cathode was swept from -1.5 V versus a standard hydrogen electrode (SHE) to 1V vs. SHE, and then back to -1.5 volts versus SHE thirty times at a scan rate of 50mV/s. The current produced during the last scan is labeled as the "argon" scan in Figure 8.

Next carbon dioxide was bubbled through the sparging tube at 200 sccm for 30 minutes, and the same scanning technique was used. That produced the CO₂ scan in Figure 8. Notice the peak starting at -0.2 volts with respect to SHE, and reaching a maximum at -0.4 V with respect to SHE. That peak is associated with CO₂ conversion.

We have also used broad-band sum frequency generation (BB-SFG) spectroscopy to look for products of the reaction. We only detect our desired product carbon monoxide in the voltage range shown (namely, the selectivity is about 100%) Oxalic acid is detected at higher potentials.

Table 1 compares these results to results from the previous literature. The table shows the actual cathode potential. More negative cathode potentials correspond to higher overpotentials. More precisely the overpotential is the difference between the thermodynamic potential for the reaction (about -0.2 V with respect to SHE) and the actual cathode potential. The values of the cathode overpotential are also given in the table. Notice that the addition of the Helper Catalyst has reduced the cathode overpotential (namely, lost work) on platinum by a factor of 4.5 and improved the selectivity to nearly 100%.

**Table 1**

| (Comparison of data in Example 1 to results reported in previous literature) | | | | |
|---|---|---|---|---|
| Reference | Catalytically Active Element | Cathode potential versus SHE | Cathode overpotential | Selectivity to carbon-containing products |
| Data from Example 1 | Platinum (+EMIM-BF₄) | -0.4 V | 0.2 V | ~100% |
| Hori Review Table 3 | Platinum (+water) | -1.07 V | 0.87 V | 0.1% |
| The Li and Oloman papers and the | Tin | -2.5 to -3.2 V | 2.3 to 3 V | 40-70% |
| application | | | | |

**Table 2**

| (Cathode potentials where CO2 conversion starts on a number of Catalytically Active Elements as reported in the Hori Review) | | | | | |
|---|---|---|---|---|---|
| Metal | Cathode potential (SHE) | Metal | Cathode potential (SHE) | Metal | Cathode potential (SHE) |
| Pb | -1.63 | Hg | -1.51 | Tl | -1.60 |
| In | -1.55 | Sn | -1.48 | Cd | -1.63 |
| Bi | -1.56 | Au | -1.14 | Ag | -1.37 |
| Zn | -1.54 | Pd | -1.20 | Ga | -1.24 |
| Cu | -1.44 | Ni | -1.48 | Fe | -0.91 |
| Pt | -1.07 | Ti | -1.60 | | |

Table 2 indicates the cathode potential needed to convert CO₂. Notice that all of the values are more negative than -0.9 V. By comparison, Figure 8 shows that CO₂ conversion starts at -0.2 V with respect to the reversible hydrogen electrode (RHE,) when the Active Element, Helper Catalyst Mixture is used as a catalyst. More negative cathode potentials correspond to higher overpotentials. This is further confirmation that Active Element, Helper Catalyst Mixtures are advantageous for CO₂ conversion.

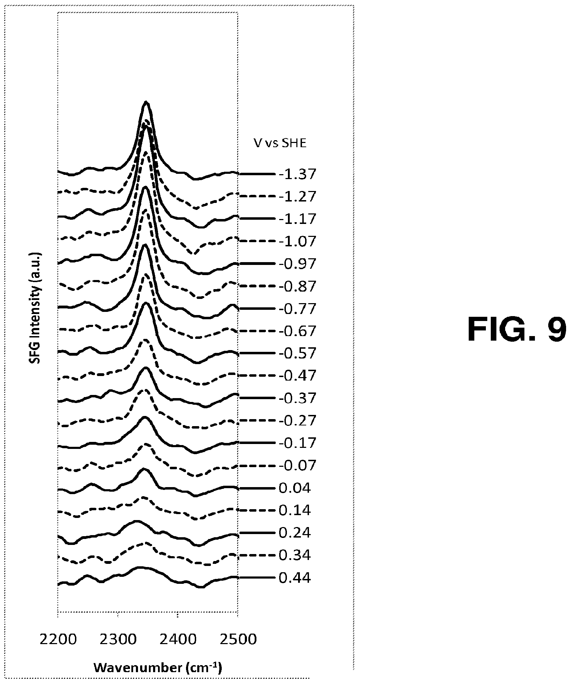

Figure 9 shows a series of BB-SFG spectra taken during the reaction. Notice the peak at 2350 cm-1. This peak corresponded to the formation of a stable complex between the Helper Catalyst and (CO₂)⁻. It is significant that the peak starts at -0.1 V with respect to SHE. According to the Hori Review, (CO₂)⁻ is thermodynamically unstable unless the potential is more negative than -1.2 V with respect to SHE on platinum. Yet Figure 9 shows that the complex between EMIM-BF4 and (CO₂)⁻ is stable at -0.1 V with respect to SHE.

Those trained in the art should recognize that this result is very significant. According to the Hori Review, the Dubois Review and references therein, the formation of (CO₂)⁻ is the rate determining step in CO₂ conversion to CO, OH-, HCO-, H₂CO, (HCO₂)-, H₂CO₂, CH₃OH, CH₄, C₂H₄, CH₃CH₂OH, CH₃COO⁻; CH₃COOH, C₂H₆, O₂, H₂, (COOH)₂, and (COO⁻)₂ on V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Sn, Tl, Pb, Bi, Sb, Te, U, Sm, Tb, La, Ce, and Nd. The (CO₂)⁻ is thermodynamically unstable at low potentials, which leads to a high overpotential for the reaction as indicated in Figure 2. The data in Figure 9 shows that one can form the EMIM- BF4-(CO₂)⁻ complex at low potentials. Thus, the reaction can follow a low energy pathway for CO₂ conversion to CO, OH⁻, HCO⁻, H₂CO, (HCO₂)⁻, H2 CO2, CH₃OH, CH₄, C₂H₄, CH₃CH₂OH, CH₃COO, CH₃COOH, C₂H₆, O₂, H₂, (COOH)₂, or (COO⁻)₂ on V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Sn, Tl, Pb, Bi, Sb, Te, U, Sm, Tb, La, Ce, and Nd as indicated in Figure 3.

In order to understand the economic consequences of this result, we calculated the cost of the electricity needed to create 100,000 metric tons per year of formic acid via two processes, (i) the process described in The Li and Oloman papers and the '727 application, and (ii) a similar process using the catalyst in this example. In both cases we assumed that the anode would run at +1.2 V with respect to SHE and that electricity would cost $0.06/kW-hr, and we scaled the current to be reasonable. The results of the calculations are given in Table 3. Notice that the calculations predict that the electricity cost will go down by almost a factor of 5 if the new catalysts are used. These results demonstrate the possible impact of the new catalysts disclosed here.

**Table 3**

| (Comparison of the projected costs using catalyst in Li and Oloman papers and the '727 application, and a similar process using the catalyst in this example) | | | | | |
|---|---|---|---|---|---|
| Catalyst | Cathode potential, V (SHE) | Anode Potential, V (SHE) | Net Potential V | Selectivity | Yearly Electricity cost |
| The Li and Oloman papers and the '727 application | -3.2 | 1.2 | 4.4 | 0.6 | $65,000,000 |
| Active Element, Helper Catalyst Mixture | -0.4 | 1.2 | 1.6 | 1 | $14,000,000 |

### Specific Example 2: The effect of dilution on the electrochemical conversion of CO₂

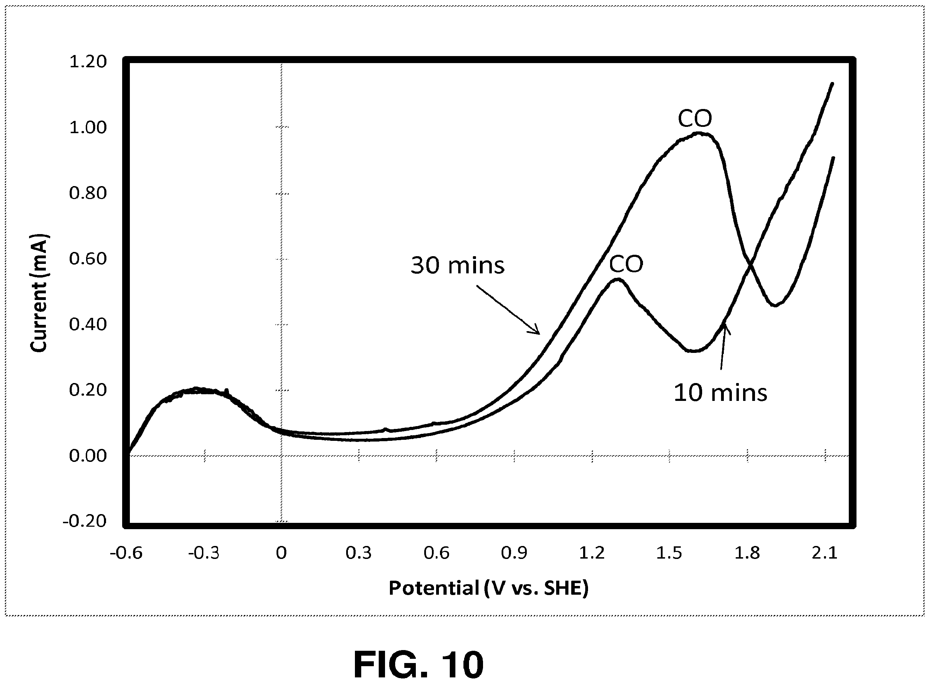

This example shows that water additions speed the formation of CO. The experiment used the Cell and procedures in Example 1, with the following exception: a solution containing 98.55% EMIM-BF4 and 0.45% water was substituted for the 99.9999% EMIM-BF4 used in Example 1, the potential was held for 10 or 30 minutes at -0.6 V with respect to RHE, and then the potential was ramped positively at 50 mV/sec. Figure 10 shows the result. Notice the peak between 1.2 and 1.5 V. This is the peak associated with CO formation and is much larger than in Example 1. Thus the addition of water has accelerated the formation of CO presumably by acting as a reactant.

### Specific Example 3 (not claimed)

Using a Active Element, Helper Catalyst Mixture including palladium and choline iodide to CO₂ lower the overpotential for electrochemical conversion of CO₂ in water.

The next example is to demonstrate that the invention can be practiced using palladium as an active element and choline iodide as a Helper Catalyst.

The experiment used the Cell and procedures in Example 1, with the following exceptions: ii) a 10.3% by weight of a Helper Catalyst, choline iodide, in water solution was substituted for the 1-ethyl-3-methylimidazolium tetrafluoroborate and ii) a 0.25 cm2 Pd foil purchased from Alfa Aesar of Ward Hill, MA, USA, was substituted for the gold plug and platinum black on the cathode, and a silver/silver chloride reference was used.

The cell contained 52 mg of palladium and 103 mg of helper catalyst, so the overall catalyst mixture contained 66% of helper catalyst.

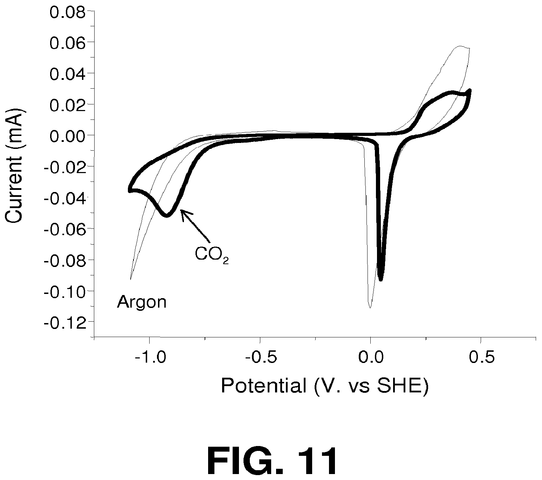

Figure 11 shows a CV taken under these conditions. There is a large negative peak near zero volts with respect to SHE associated with iodine transformations and a negative going peak at about 0.8 V associated with conversion of CO₂. By comparison the data in Table 2 indicates that one needs to use a voltage more negative that -1.2 V to convert CO₂ on palladium in the absence of the Helper Catalyst. Thus, the Helper Catalyst has lowered the overpotential for CO₂ formation by about 0.5 V.

This example also demonstrates that the invention can be practiced with a second Active Element, palladium, and a second Helper Catalyst, choline iodide. Further, those trained in the state of the art will note that there is nothing special about the choice of palladium and choline iodide. Rather, this example shows that the results are general and not limited to the special case in Example 1.

### Specific Example 4 (not claimed)

Using an Active Element, Helper Catalyst Mixture that includes palladium and choline chloride to lower the overpotential for electrochemical conversion of CO₂ to formic acid.

The next example is to demonstrate that the invention can be practiced using a third Helper Catalyst, choline chloride.

The experiment used the Cell and procedures in Example 3, with the following exception: a 6.5% by weight choline chloride in water solution was substituted for the choline iodide solution.

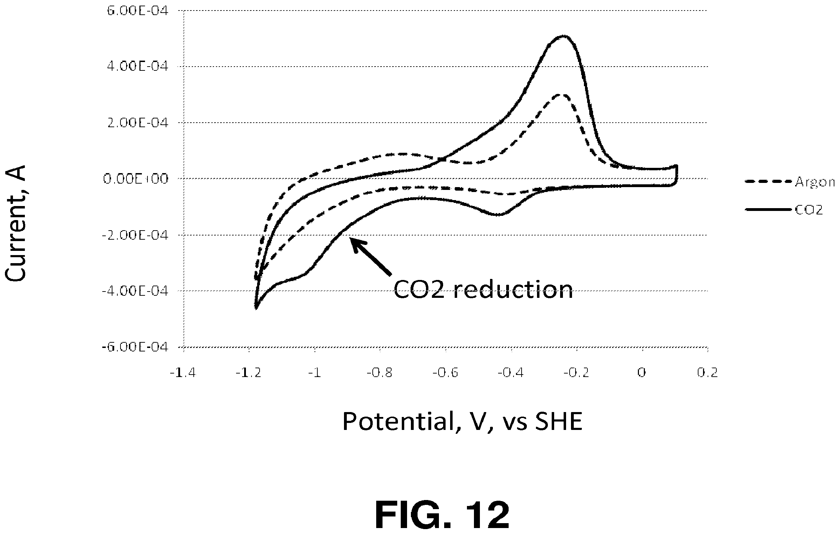

The cell contained 52 mg of palladium and 65 mg of helper catalyst, so the overall catalyst mixture contained 51% of helper catalyst. Figure 12 shows a comparison of the cyclic voltammetry for a blank scan where i) the water-choline chloride mixture was sparged with argon and ii) a scan where the water-choline chloride mixture was sparged with CO₂. Notice the negative going peaks starting at about -0.6. This shows that CO₂ is being reduced at -0.6 V. By comparison the data in Table 2 indicates that a voltage more negative than -1.2 V is needed to convert CO₂ on palladium in the absence of the Helper Catalyst. Thus, the overpotential for CO₂ conversion has been lowered by 0.6 V by the Helper Catalyst.

Another important point is that there is no strong peak for hydrogen formation. A bare palladium catalyst would produce a large hydrogen peak at about - 0.4 V at a pH of 7, while the hydrogen peak moves to -1.2 V in the presence of the Helper Catalyst. The Hori Review reports that palladium is not an effective catalyst for CO₂ reduction because the side reaction producing hydrogen is too large. The data in Figure 12 show that the Helper Catalysts are effective in suppressing hydrogen formation.

We have also used CV to analyze the reaction products. Formic acid was the only product detected. By comparison, the Hori Review reports that the reaction is only 2.8% selective to formic acid in water. Thus the Helper Catalyst has substantially improved the selectivity of the reaction to formic acid.

This example also demonstrates that the invention can be practiced with a third Helper Catalyst, choline chloride. Further, those trained in the state of the art will note that there is nothing special about the choice of palladium and choline chloride. Rather, this example shows that the results are general and not limited to the special case in Example 1.

Further, those trained in the state of art should recognize that the results should not depend on the thickness of the palladium foil. For example if we increase the thickness of the palladium foil by a factor of 10, the active element-helper catalyst mixture would only contain 11% of helper catalyst. If the foil thickness is increased to 0.5 inches, the mixture will contain about 1% of helper catalyst.

### Specific Example 5 (not claimed)

Using an Active Element, Helper Catalyst Mixture that includes nickel and choline chloride to lower the overpotential for electrochemical conversion of CO₂ to CO.

The next example is to demonstrate that the invention can be practiced using a third metal, nickel.

The experiment used the Cell and procedures in Example 4, with the following exception: a nickel foil from Alfa Aesar was substituted for the palladium foil.

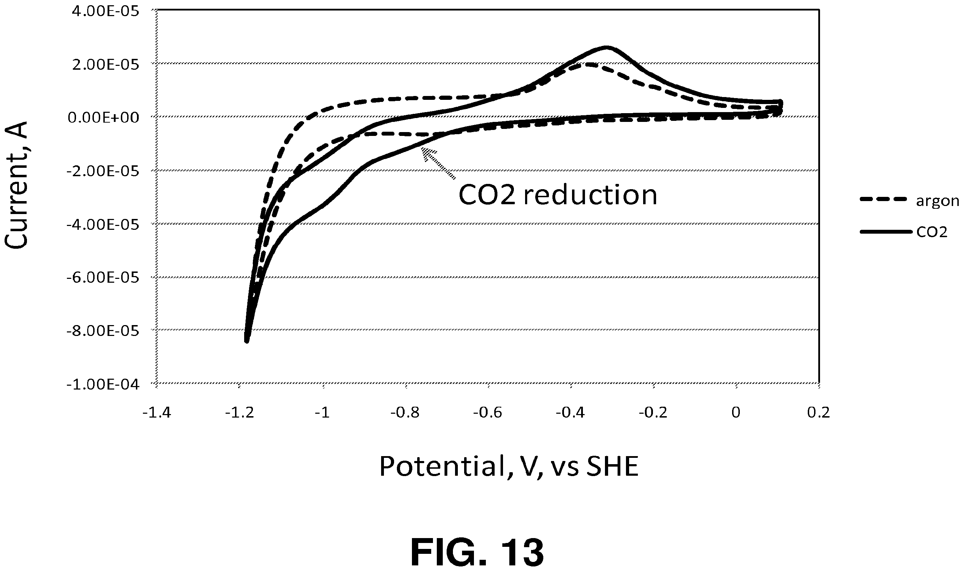

Figure 13 shows a comparison of the cyclic voltammetry for a blank scan where i) the water-choline chloride mixture was sparged with argon and ii) a scan where the water-choline chloride mixture was sparged with CO₂. Notice the negative going peaks starting at about -0.6. This shows that CO₂ is being reduced at -0.6 V. By comparison, the data in Table 2 indicates that a voltage more negative than -1.48 V is needed to convert CO₂ on nickel in the absence of the Helper Catalyst. Thus, the Helper Catalyst has lowered the overpotential for CO₂ conversion.

Another important point is that there is no strong peak for hydrogen formation. A bare nickel catalyst would produce a large hydrogen peak at about -0.4 V at a pH of 7, while the hydrogen peak moves to -1.2 V in the presence of the Helper Catalyst. The Hori Review reports that nickel is not an effective catalyst for CO₂ reduction because the side reaction producing hydrogen is too large. The data in Figure 13 show that the Helper Catalysts are effective in suppressing hydrogen formation.

Also the Helper Catalyst is very effective in improving the selectivity of the reaction. The Hori Review reports that hydrogen is the major product during carbon dioxide reduction on nickel in aqueous solutions. The hydrolysis shows 1.4% selectivity to formic acid, and no selectivity to carbon monoxide. By comparison, analysis of the reaction products by CV indicates that carbon monoxide is the major product during CO₂ conversion on nickel in the presence of the Helper Catalyst. There may be some formate formation. However, no hydrogen is detected. This example shows that the Helper Catalyst has tremendously enhanced the selectivity of the reaction toward CO and formate.

This example also demonstrates that the invention can be practiced with a third metal, nickel. Further, those trained in the state of the art will note that there is nothing special about the choice of nickel and choline chloride. Rather, this example shows that the results are general and not limited to the special case in Example 1.

Those trained in the state of art should realize that since choline chloride and choline iodide are active, other choline salts such as choline bromide, choline fluoride and choline acetate should be active too.

### Specific Example 6

Demonstration that an Active Element (Gold), Helper Catalyst Mixture is useful in a CO₂ sensor.

This example demonstrates that the invention can be practiced with a fourth Active Element, gold. It also demonstrates that the catalysts are useful in sensors.

The sensor may be a simple electrochemical device wherein an Active Element, Helper Catalyst Mixture is placed on an anode and cathode in an electrochemical device, then the resistance of the sensor is measured. If there is no CO₂ present, the resistance will be high, but preferably not infinite, because of leakage currents. When CO₂ is present, the Active Element, Helper Catalyst Mixture may catalyze the conversion of CO₂. That allows more current to flow through the sensor. Consequently, the sensor resistance decreases. As a result, the sensor may be used to detect carbon dioxide.

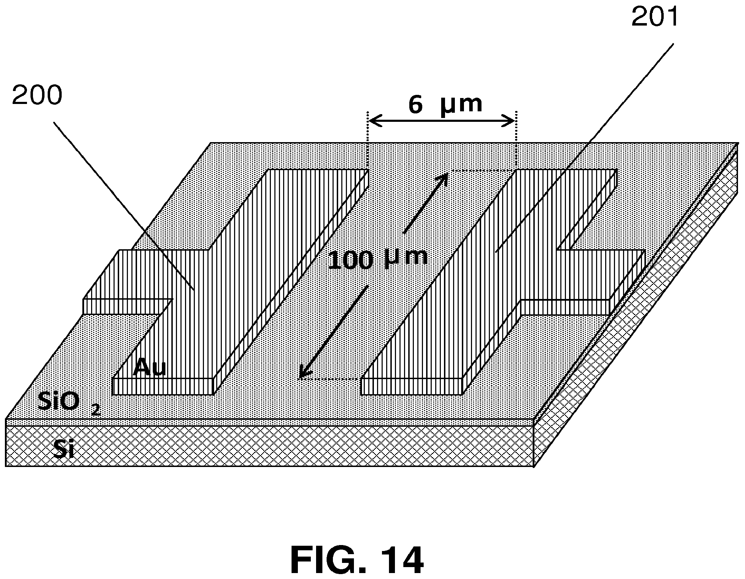

An example sensor was fabricated on a substrate made from a 100 mm silicon wafer (Silicon Quest International, Inc., Santa Clara, CA, USA, 500 pm thick, <100> oriented, 1-5 Ω·cm nominal resistivity) which was purchased with a 500 nm thermal oxide layer. On the wafer, 170 A chromium was deposited by DC magnetron sputtering (~ 10-2 Torr of argon background pressure). Next, 1000 Å of a Catalytically Active Element, gold, was deposited on the chromium and the electrode was patterned via a standard lift-off photolithography process to yield the device shown schematically in Figure 14.

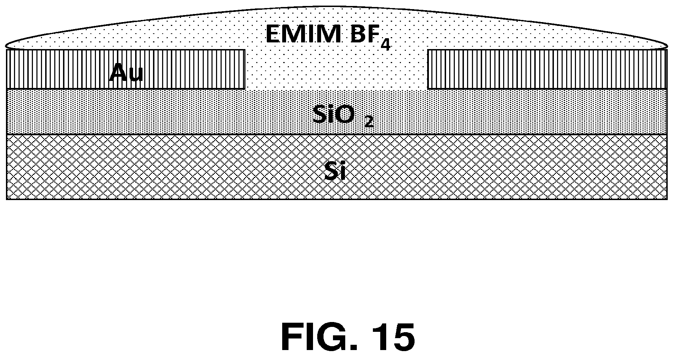

At this point, the device consisted of an anode (200) and cathode (201) separated by a 6 µm gap, [Note: Figs. 14 and 15 do not include the reference numerals 200, 201, 202 or the mu symbol for µm.] wherein the anode and cathode were coated with a Catalytically Active Element, gold. At this point the sensor could not detect CO₂.

Next 2µl of a Helper Catalyst, EMIM BF4 (202) was added over the junction as shown in Figure 15. The device was mounted into a sensor test cell with wires running from the anode and cathode.

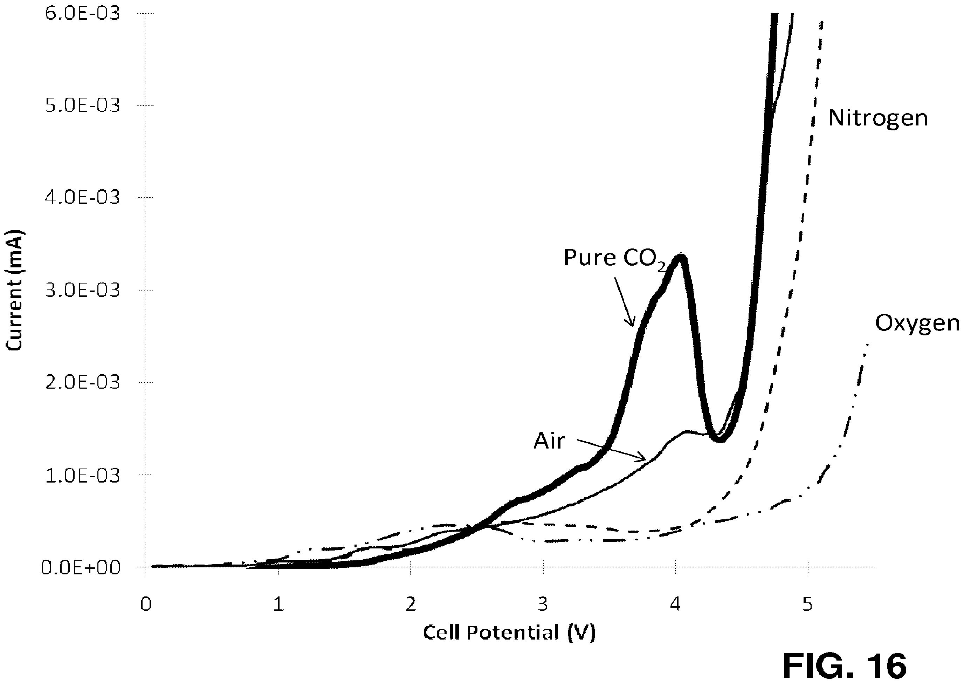

Next, the anode and cathode were connected to a SI 1287 Solartron electrical interface, and the catalysts were condition by sweeping from 0 volts to 5 volts at 0.1 V/sec and then back again. The process was repeated 16 times. Then the sensor was exposed to either nitrogen, oxygen, dry air or pure CO₂, and the sweeps were recorded. The last sweep is shown in Figure 16. Notice that there is a sizable peak at an applied voltage of 4 volts in pure CO2. That peak is associated with the electrochemical conversion of CO₂.

Notice that the peak is absent when the sensor is exposed to oxygen or nitrogen, but it is clearly seen when the sensor is exposed to air containing less than 400 ppm of CO₂. Further the peak grows as the CO₂ concentration increases. Thus, the sensor can be used to detect the presence of CO₂.

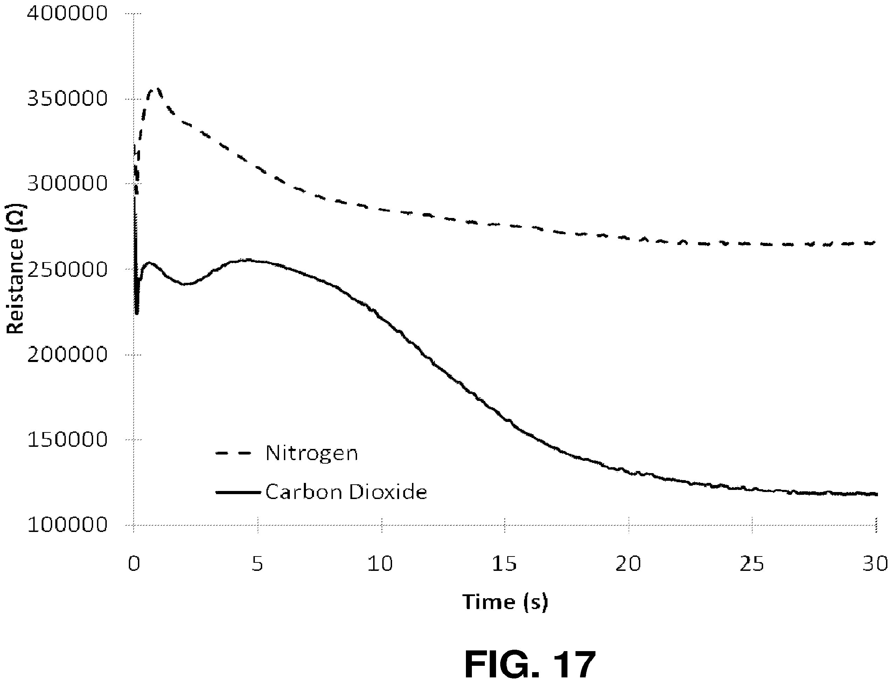

We have also run the sensor in a galvanastatic mode, where we measured the voltage needed to maintain the current constant at 1 microamp, and measured the voltage of the device. Figure 17 shows that less voltage is needed to maintain the current when CO₂ is added to the cell. This shows that the sensor that includes an Active Element, Helper Catalyst Mixture responds to the presence of CO₂.

Table 4 compares the sensor here to those in the previous literature. Notice that the new sensor uses orders of magnitude less energy than commercial CO₂ sensors. This is a key advantage for many applications.

This example also illustrates that the invention can be practiced with a fourth Active Element, gold.

**Table 4**

| (Comparison of power needed to run the present sensor to that needed to operate commercially available CO2 sensors) | | | |
|---|---|---|---|
| Sensor | Power | Sensor | Power |
| Specific Example 6 | 5x10-7 watts | GE Ventostat 8100 | 1.75 watts |
| Honeywell C7232 | 3 watts | Vaisala CARBOCAP GMP343 | about 1 watt |

### Specific Example 7

This example illustrates steady state production of carbon monoxide.

This experiment used the flow cell described in . T. Whipple, E. C. Finke, and P. J. A. Kenis, Electrochem. & Solid-State Lett., 2010, 13 (9), B109-B111. (the Whipple paper) First catalyst inks were prepared as follows:

For the cathode: 10 mg of silver nanoparticles (Sigma Aldrich) was sonicated into a solution containing 100 µL or water, 100 µL of isopropyl alcohol and 5.6 µL of 5% Nafion (perfluorosulfonic acid) solution (Ion Power). The resultant catalyst ink was painted on a 1x1.5 cm section of a 2x3 cm piece of carbon paper (ion power) and dried with a heat lamp.

The preparation was identical for anode except 4 mg of HiSpec 1000 platinum black (Sigma Adrich) was substituted for the silver.

Both catalysts were mounted in the flow cell described in the Whipple Paper. Five seem of CO₂ was fed to the anode, and a solution containing 18 mole percent of EMIM-BF4 in water was fed into the gap between the anode and the cathode. At any one time the cell contained approximately 10 mg of silver nanoparticles and approximately 40 mg of EMIM-BF4 helper catalyst. A potential was applied to the cell, and the data in Table 4 were obtained. This results demonstrates that steady state production of useful products can be obtained with Catalytically Active Element-Helper Catalyst Mixtures

**Table 4**

| (Products produced at various conditions) | | |
|---|---|---|
| Cathode potential vs. RHE | Hydrogen production rate, µg/min | Carbon monoxide production rate, |
| -0.358 | 0 | 0 |
| -0.862 | 1.1 | 2.6 |
| -1.098 | 1.4 | 50 |
| -1.434 | 1.1 | 250 |
| -1.788 | 0 | 560 |

## Claims

1. A process for making a chemical reaction product, comprising the steps of:
combining a catalytically active element selected from the group consisting of V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Tl, Pb, Bi, Sb, Te, U, Sm, Tb, La, Ce and Nd and a helper catalyst in the presence of a reactant comprising CO₂, said helper catalyst comprising 1-ethyl-3-methylimidazonium cations, wherein said helper catalyst further comprises tetrafluoroborate anions;
allowing a reaction to proceed to produce a reaction product, optionally comprising the step of:
applying electrical energy to the reaction so as to achieve electrochemical conversion of the reactant to a reaction product.

2. The process of claim 1 wherein said catalytically active element is selected from the group consisting of Pb, Hg, Tl, In, Cd, Bi, Zr, Cr, Sn, W, Pd and Ru.

3. The process of claim 1 wherein said reaction product is at least one of CO, HCO⁻, H₂CO, (HCO₂)⁻, H₂CO₂, CH₃OH, CH₄, C₂H₄, CH₃CH₂OH, CH₃COO⁻, CH₃COOH, C₂H₆, (COOH)₂, and (COO⁻)₂, wherein said reaction product preferably is carbon monoxide (CO) or formic acid (H₂CO₂).

## Patentansprüche

1. Verfahren zur Herstellung eines chemischen Reaktionsprodukts, umfassend die Schritte:
Kombinieren eines katalytisch aktiven Elements, ausgewählt aus der Gruppe bestehend aus V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Tl, Pb, Bi, Sb, Te, U, Sm, Tb, La, Ce und Nd, und eines Hilfskatalysators in Gegenwart eines CO₂ umfassenden Reaktanten, wobei der Hilfskatalysator 1-Ethyl-3-Methylimidazonium-Kationen umfasst, wobei der Hilfskatalysator ferner Tetrafluoroborat-Anionen umfasst;
Ablaufen lassen einer Reaktion, um ein Reaktionsprodukt zu erzeugen, optional umfassend den Schritt des:
Anlegens von elektrischer Energie an die Reaktion, um eine elektrochemische Umwandlung des Reaktanten in ein Reaktionsprodukt zu erreichen.

2. Verfahren nach Anspruch 1, wobei das katalytisch aktive Element ausgewählt ist aus der Gruppe bestehend aus Pb, Hg, Tl, In, Cd, Bi, Zr, Cr, Sn, W, Pd und Ru.

3. Verfahren nach Anspruch 1, wobei das Reaktionsprodukt mindestens eines ist aus CO, HCO⁻, H₂CO, (HCO₂)⁻, H₂CO₂, CH₃OH, CH₄, C₂H₄, CH₃CH₂OH, CH₃COO⁻, CH₃COOH, C₂H₆, (COOH)₂ und (COO⁻)₂, wobei das Reaktionsprodukt vorzugsweise Kohlenmonoxid (CO) oder Ameisensäure (H₂CO₂) ist.

## Revendications

1. Procédé de fabrication d'un produit réactionnel chimique, comprenant les étapes consistant à:
combiner un élément catalytiquement actif sélectionné dans le groupe constitué par V, Cr, Mn, Fe, Co, Ni, Cu, Sn, Zr, Nb, Mo, Ru, Rh, Pd, Ag, Cd, Hf, Ta, W, Re, Ir, Pt, Au, Hg, Al, Si, In, Tl, Pb, Bi, Sb, Te, U, Sm, Tb, La, Ce et Nd et un catalyseur auxiliaire en présence d'une charge comprenant du CO2, ledit catalyseur auxiliaire comprenant des actions de 1-éthyl-3-méthylimidazonium, dans lequel ledit catalyseur auxiliaire comprend en outre des anions de tétrafluoroborate;
permettre une réaction pour procéder à la production d'un produit réactionnel, éventuellement comprenant l'étape consistant à:
appliquer de l'énergie électrique à la réaction de manière à obtenir une conversion électrochimique de la charge en un produit réactionnel.

2. Procédé selon la revendication 1, dans lequel ledit élément catalytiquement actif est sélectionné dans le groupe constitué par Pb, Hg, Tl, In, Cd, Bi, Zr, Cr, Sn, W, Pd et Ru.

3. Procédé selon la revendication 1, dans lequel ledit produit réactionnel est au moins l'un parmi CO, HCO⁻, H₂CO, (HCO₂)⁻, H₂CO₂, CH₃OH, CH₄, C₂H₄, CH₃CH₂OH, CH₃COO⁻, CH₃COOH, C₂H₆, (COOH)₂, et (COO⁻)₂, dans lequel ledit produit réactionnel est de préférence du monoxyde de carbone (CO) ou de l'acide formique (H₂CO₂).
